# EUROPEAN PATENT SPECIFICATION

(11) **EP 3 127 546 B1**
(45) Date of publication and mention of the grant of the patent: **01.07.2020**
(21) Application number: 16183010.4
(22) Date of filing: 05.08.2016
(51) Int. Cl.: A61K 33/06, A61K 35/74, A61P 29/02, A61P 43/00

(54) **REDUCTION OF OXALATE ABSORPTION IN INDIVIDUALS**
REDUKTION VON OXALSÄUREABSORPTION BEI PERSONEN
RÉDUCTION D'ABSORPTION D'OXALATE CHEZ DES INDIVIDUS

(30) Priority: 06.08.2015 US 201562201842 P; 04.08.2016 US 201615227995
(43) Date of publication of application: 08.02.2017
(73) Proprietor: Fine, Kenneth Davin, Dallas, TX 75238 (US)
(72) Inventor: Fine, Kenneth Davin, Dallas, TX 75238 (US)
(74) Representative: Potter Clarkson

(56) References cited:
- EP-A1- 1 338 284
- EP-A2- 0 150 792
- WO-A1-97/38701
- WO-A1-2015/002588
- US-A1- 2006 029 641
- US-A1- 2014 271 929
- LIEBMAN ET AL: "EFFECTS OF CALCIUM AND MAGNESIUM ON URINARY OXALATE EXCRETION AFTER OXALATE LOADS", JOURNAL OF UROLOGY, LIPPINCOTT WILLIAMS & WILKINS, BALTIMORE, MD, US, vol. 163, no. 5, 1 May 2000 (2000-05-01), pages 1565-1569, XP005555471, ISSN: 0022-5347, DOI: 10.1016/S0022-5347(05)67680-X
- ABRATT VALERIE R ET AL: "Oxalate-Degrading Bacteria of the Human Gut as Probiotics in the Management of Kidney Stone Disease", ADVANCES IN APPLIED MICROBIOLOGY, ACADEMIC PRESS, UNITED STATES, vol. 72, 1 January 2010 (2010-01-01), pages 63-87, XP009192500, ISSN: 0065-2164

## Description

### TECHNICAL FIELD

The present invention relates to orally administered supplements for use in reducing fibromyalgia pain in a mammal and/or reducing pain due to calcification of the spine and/or a shoulder of a mammal.

### BACKGROUND

Dietary habits change and with these changes unanticipated impacts on an individual's health may occur. For example, as more vegetable centric and/or raw food diets are adopted by greater proportions of the population, individuals, with or without predispositions, may experience improved health in certain ways but experience new complications in other ways that may diminish overall health. For example, consumption of leafy greens and other mostly plant-based foods rich in oxalate may increase the absorption of oxalate in the body. Rather than decrease the amount of healthy, yet oxalate rich, foods than an individual may consume, there is a need for a way to decrease oxalate absorption.

### SUMMARY

as the present invention provides an oxalate absorption blocking (OAB) composition comprising 100 mg to 2500 mg calcium and 50 mg to 1500 mg magnesium for use in reducing fibromyalgia pain in a mammal and/or reducing pain due to calcification of the spine and/or a shoulder of a mammal, wherein a therapeutically effective amount of the oxalate absorption blocking composition is for oral administration. The OAB composition may be granular, liquid, and/or another appropriate form that may be orally administered. In some implementations, the OAB composition may include carbonate(s), probiotic(s), vitamin(s), and/or other mineral(s). The oral administration of the OAB composition may decrease the amount of oxalate absorbed, by the body of an individual, from foods consumed by the individual.

In various implementations, health may be improved in an individual by orally administering a therapeutically effective amount of an oxalate absorption blocking composition. The pain may be reduced by reducing the amount of oxalate absorbed by the individual from consumed meals. The health of the individual may be improved (e.g., when compared with an individual not being administered the oxalate absorbing composition) by reducing the amount of oxalate absorbed by the individual from consumed meals.

Implementations may include one or more of the following features. The oxalate absorption blocking composition comprises one or more probiotics. The oxalate absorption blocking composition comprises *Oxalobacter formigenes.* An atomic ratio of calcium to magnesium in the oxalate absorption blocking composition comprises approximately 1 to approximately 2 Calcium: to approximately 1 Magnesium. The oxalate absorption blocking composition is administered at least twice a day. The oxalate absorption blocking composition is administered proximate consumption of a meal. The oxalate absorption blocking composition comprises approximately 100 mg of Calcium to approximately 2500 mg of Calcium, and wherein the oxalate absorption blocking composition comprises approximately 50 mg of Magnesium to approximately 1500 mg of Magnesium. In some implementations, the oxalate absorption blocking composition comprises approximately 800 mg of Calcium to approximately 1000 mg of Calcium, and wherein the oxalate absorption blocking composition comprises approximately 200 mg of Magnesium to approximately 300 mg of Magnesium. In some implementations, improving a health of an individual comprises at least one of reducing symptoms of a congenital disease related to hyper-absorption of oxalate by reducing oxalate absorption during consumption of meals, reducing symptoms of hyperoxaluria, reducing frequency of kidney stones, reducing size of kidney stones, reducing gout flare ups, reducing fibromyalgia pain, reducing pain of rheumatoid arthritis, reducing symptoms of vulvodynia, reducing symptoms of calcification of the spine, and/or reducing symptoms of calcification of the shoulder by reducing an amount of oxalate absorbed by the mammal during the consumption of meals.

In various implementatations, oxalate absorption may be reduced in a mammal by orally administering a therapeutically effective amount of an oxalate absorption blocking composition proximate consumption of a meal.

The oxalate absorption blocking composition may be administered at least twice a day. The oxalate absorption blocking composition may comprise one or more probiotics.

Implementations may include one or more of the following features. The oxalate absorption blocking composition comprises one or more probiotics. The oxalate absorption blocking composition comprises an analgesic. The oxalate absorption blocking composition comprises approximately 800 mg of Calcium to approximately 1000 mg of Calcium. The oxalate absorption blocking composition comprises approximately 200 mg of Magnesium to approximately 300 mg of Magnesium. The oxalate absorption blocking composition is administered at least twice a day.

The details of one or more implementations are set forth in the accompanying drawings and the description below. Other features, objects, and advantages of the implementations will be apparent from the description and drawings.

### BRIEF DESCRIPTION OF THE DRAWINGS

For a more complete understanding of this disclosure and its features, reference is now made to the following description, taken in conjunction with the accompanying drawings, in which:

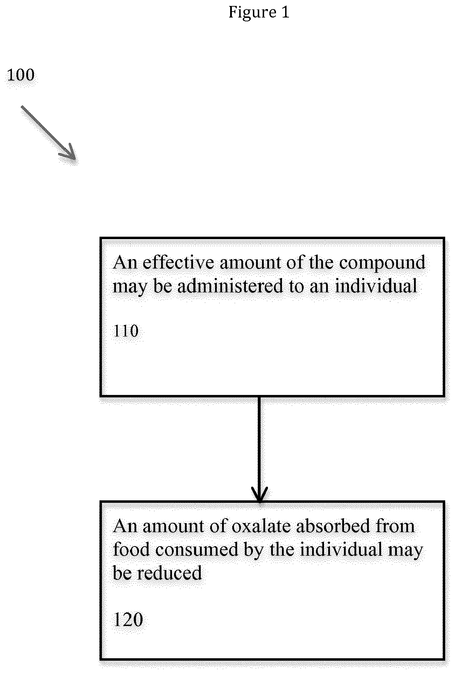

Figure 1 illustrates an implementation of an example process for administering a supplement.

### DETAILED DESCRIPTION

Oxalate is present in a variety of foods including leafy green vegetables; root vegetables such carrots and beets; grains, such as rice and wheat bran; beverages, such as tea, coffee, smoothies, and juices; fruits including blueberries, strawberries and apples; candy; and other foods. Some foods include high levels of oxalate such as greens (e.g., spinach and beet greens), rhubarb, beets, nuts, chocolate, tea, coffee, soybeans, wheat bran, and strawberries.

Some individuals may be predisposed to hyper-absorb oxalate (e.g., due to congenital diseases, genetic predisposition, pathologic induction of increased intestinal permeability from inflammatory processes, and/or due to natural variations in humans). Some individuals may have hyperoxaluria (e.g., high oxalate levels in their urine) due to a variety of reasons. For example, an individual may lack an enzyme or certain strains of intestinal bacteria in the gastrointestinal tract that break down oxalate; and/or an individual may have dietary practices that cause hyperoxaluria (e.g., consumption of large quantities of foods high in oxalate, diet high in fat, diet low in calcium, etc.). In some individuals, medical procedures, such as when a portion of the intestine has been removed, may cause greater sensitivity to oxalates (e.g., due to its hyperabsorption) than an individual had experienced previously.

Excess oxalate absorption may cause negative health effects in some individuals. For example, excess oxalate in an individual (e.g., as measured by increased oxalate levels in the urine, blood, or other appropriate measurement) may be associated with kidney stones and/or gout. Excess oxalate absorption in an individual may increase fibromyalgia pain. Excess oxalate in an individual may increase and/or cause pain due to vulvodynia, rheumatoid arthritis, and/or calcification of the spine (e.g., rheumatic spondyloarthropathies) and/or shoulder (e.g., frozen shoulder syndrome).

Thus, an OAB composition that can be administered to reduce the absorption of oxalate from food consumed by an individual may inhibit absorption of at least a portion of the oxalate in consumed foods and/or maintain the health of the individual (e.g., by reducing and/or inhibiting pain, kidney stones, gout symptoms, etc.) In addition, rather than limit the consumption of healthy oxalate foods, an individual may be able to benefit from the healthy foods while inhibiting the negative affects of excess oxalate absorption by the individual's body. Thus, some individuals may benefit from the administration of a therapeutically effective amount of an OAB composition to decrease oxalate absorption, by the body, from foods consumed by an individual.

The OAB composition includes calcium and magnesium. For example, the OAB composition may include dolomite, a natural occurring mineral containing these salts. In some implementations, the OAB composition may include one or more calcium OAB compositions and one or more magnesium OAB compositions. For example, the OAB composition may include predetermined combinations of calcium carbonate and/or magnesium carbonate (e.g., isolated calcium carbonate and/or isolated magnesium carbonate). The OAB composition may include a predetermined ratio of calcium to magnesium (e.g., approximately 1 Ca: approximately 1 Mg, approximately 2 Ca: approximately 1 Mg, approximately 2.5 Ca: approximately 1 Mg and/or any other appropriate atomic ratio).

The predetermined ratio of calcium to magnesium may be based on a therapeutically effective amount for the reduction of absorption of oxalate from consumed foods. The predetermined ratio of calcium to magnesium may be based on inhibiting side effects, such as laxative effects. In some implementations, the predetermined ratio of calcium to magnesium may be based on stimulating a laxative effect to be used by individuals who ordinarily tend towards constipation. For example, the therapeutically effective amount of the OAB composition to be administered may be an atomic ratio of approximately 1 to 2 of calcium and approximately 1 of magnesium (e.g., a atomic ratio ranges from approximately 1: approximately 1 to approximately 2: approximately 1). The therapeutically effective amount of the OAB composition to be administered may be an atomic ratio of approximately 1 to 3 of calcium and approximately 1 of magnesium. Thus, in one preparation, the therapeutically effective amount may not cause substantial gastrointestinal problems such an amount that would provide a laxative effect, while another might be formulated to provide a gentle desired laxative effect.

In some implementations, the OAB composition may include carbonate(s), vitamin(s), and/or other mineral(s). In some implementations, the carbonate(s), vitamin(s), and/or other mineral(s) may include calcium and/or magnesium. For example, the OAB composition may include calcium carbonate. In some implementations, the OAB composition may include a vitamin, such as B6. The OAB composition may include additives such as flavorings, dyes, preservatives, etc, in some implementations.

In some implementations, the OAB composition may include an analgesic. For example, the OAB may include acetaminophen and/or nonsteroidal anti-inflammatory drug (NSAID) (e.g., aspirin, ibuprofen, etc.). The analgesic may provide temporary pain relief and/or anti-inflammatory properties. Since the effects of OAB administration may take time for an individual to receive (e.g., as opposed to the more immediate nature of the analgesic), administration of the analgesic via the OAB composition may supplement the pain relief provided by the reduced oxalate absorption by the OAB composition. In some implementations, an individual may initially be administered OAB with an analgesic and after a predetermined period (e.g., 1 week, 2 weeks, 1 month, etc.) be switched to administration of an OAB without analgesic compounds. In some implementations, an individual may be administered OAB with analgesics during at least one first dosage administration and may be administered OAB compound without analgesics during at least one second dosage administration (e.g., to inhibit over dosage of analgesics, to reduce an amount of administered analgesic, etc.).

In some implementations, the OAB composition may include one or more probiotics. The probiotics may include one or more non-specific, commonly administered probiotic organisms associated with healthy intestinal flora such as *Lactobacillus, Bifidobacterium,* or non-pathogenic *Streptococcu* organisms. Additionally or alternatively, the probiotics may include any appropriate specific probiotic organism, such as *Oxalobacter formigenes.* In some implementations, one or more of the probiotics selected for inclusion in the OAB composition may degrade and/or inhibit absorption of oxalate. For example, a probiotic organism such as *Oxalobacter formigenes,* may reduce the levels of oxalate absorbed by the body since *Oxalobacter formigenes* may degrade oxalate (e.g., in the presence of oxalate, such as in the intestinal tract of an individual). Administering the probiotic may promote the growth of healthy intestinal flora in the gastrointestinal tract of an individual. For example, by promoting the growth of *Oxalobacter formigenes,* and/or the other probiotic genera in the gastrointestinal tract of an individual, the amount of oxalate absorbed by the body may be decreased (e.g., since the *Oxalobacter formigenes* and/or other probiotic organisms may degrade at least a portion of the oxalate present in the gastrointestinal tract). In some implementations, the individual may decrease, stop, and/or switch types of OAB compositions administered due to the promotion of healthy flora in the gastrointestinal tract. For example, an individual may switch from an OAB composition including probiotics to an OAB composition that does not include probiotics. Switching types of OAB compositions may decrease administration costs (e.g., since the OAB composition without probiotics may be less expensive). In some implementations, the use of the OAB composition may be for a period of time to promote the growth of healthy of intestinal flora in the gastrointestinal tract. The amount of OAB composition may be decreased (e.g., to a lower dosage, to a less frequent administration schedule, and/or stopped) based on monitoring (e.g., of oxalate levels, for example, measured in the urine; of intestinal flora; and/or of symptoms, such as decreased pain and/or inflammation). In some implementations, as healthy flora increases in the gastrointestinal tract, the amount of oxalate absorbed by the individual (e.g., the gastrointestinal tract of the individual) may be decreased (e.g., when compared to the same individual prior to adminsteration of the OAB composition) and thus the administration amount may be decreased.

In some implementations, probiotic organisms in the OAB composition may promote the growth, in the gastrointestinal tract of an individual, of healthful bacteria (e.g., probiotic bacteria) over less healthful bacteria (e.g., that cause diarrhea and/or other irritation and/or inflammation of the gastrointestinal tract) and/or in individuals with less bacteria growth (e.g., due to antibiotic use). For example, the probiotics in the OAB composition may include live or dormant healthful bacteria that when present in internal body cavities of the body (e.g., intestinal tract, oral cavity, and/or vaginal cavity) have been proven to overcome the growth of less healthful bacteria (e.g., anaerobic bacteria) and/or yeast.

In some implementations, the probiotics in an OAB composition may promote the growth of bacteria, in an individual's gastrointestinal tract, that promotes an individual's health. In some implementations, at least a portion of a desiccated dormant probiotic may be revived by rehydration from the saliva present in the individual's mouth, fluids in the gastrointestinal tract, and/or fluids in consumed foods. As the probiotics in the gastrointestinal tract or portions thereof grow, other bacteria present in the area may exist in lower magnitudes and/or die (e.g., the colony may enter the death phase). When the previously existing bacteria are in lower magnitudes and/or die, an individual's health (e.g., the probability of pain, kidney stones, gout symptoms, and/or other diseases and disorders related to excess oxalate absorption) may be improved (e.g., when compared with individuals in which the OAB composition has not been administered).

In various implementations, the probiotic may include one or more predetermined strains of bacteria (e.g. *Lactobacillus species, Bifidobacterium species, non-pathogenic Streptococci, Oxalobacter formigenes*). In some implementations, the organisms (e.g., bacteria) in the probiotic may be live, dried (e.g., freeze-dried), and/or combinations thereof. The organisms in the probiotic may be similar or identical to one or more of the bacteria used in intestinal probiotic preparations, such as the previously mentioned *Lactobacillus, Bifidobacterium,* and *Streptococci* species.

In some implementations, the OAB composition may include a dried probiotic (e.g., freeze dried). The dried probiotic may include dried bacteria. The dried bacteria may include dormant or inert bacteria. By utilizing dried probiotics, the shelf life of the probiotics in the OAB composition may be increased (e.g., when compared to using live probiotic). When the probiotics are utilized in live form in the OAB composition, the number of probiotic organisms may decrease overtime (e.g., due to reduction in nutrients available or other reason for entry into a death phase of a colony of organisms). Thus, by utilizing dried probiotics (e.g., bacteria), the number of probiotic organisms (e.g., bacteria) in the OAB composition may be maintained at a higher level over time than when utilizing live probiotics.

When dried probiotics are utilized in the OAB composition, at least a portion of the dried probiotics may be activated (e.g., revived) prior to and/or during administration of the OAB composition. For example, at least a portion of the dried probiotic may be hydrated prior to administration in an individual (e.g., mixed with a hydrating agent, such as water or juice; and/or mixing with other foods, such as soup, sauce, etc.). In some implementations, at least a portion of the dried probiotic may be hydrated by the saliva present in the user's mouth and/or gastrointestinal tract. The dried probiotic may be hydrated by fluid present in the foods to be consumed by the individual (e.g., when the probiotic is sprinkled or otherwise applied to food to be consumed). When the dried probiotic is activated (e.g., revived), the probiotic may become live probiotic. For example, when dried bacteria in a dried probiotic are activated, at least a portion of the dried bacteria may be revived (e.g., returned to a live state). When the probiotic in the OAB composition is administered to an individual, the revived bacteria may improve and/or maintain healthy flora in the individual's gastrointestinal tract.

In some implementations, the other components (e.g., vitamins, flavorings, etc.) selected for inclusion in the OAB composition may not substantially inhibit growth of the probiotic in the OAB composition and/or an individual's gastrointestinal tract.

In some implementations, the OAB composition may include dolomite and a probiotic. For example, the OAB composition may include dolomite and *Oxalobacter formigenes.* In some implementations, the OAB composition may include a probiotic and not include dolomite. The OAB composition may include a probiotic and either calcium or magnesium. In some implementations, the OAB composition may include probiotics and may not include calcium and/or magnesium. The ratio of calcium to magnesium to probiotic (e.g., 1:1:0.5, 1:1:0, 2:1:0, 2:1:1, 0:0:1 and/or any other appropriate ratio) may be selected to be a therapeutically effective amount for administration to an individual. In some implementations, the OAB may include calcium carbonate, magnesium carbonate and/or a probiotic (e.g., approximately 8 Calcium carbonate: approximately 2 Magnesium carbonate: approximately 1 probiotic by weight to approximately 10 Calcium carbonate: approximately 3 Magnesium carbonate: approximately 3 probiotic by weight; approximately 8 Ca: approximately 2 Mg : 1 probiotic to approximately 10 Ca: approximately 3 Mg: approximately 3 probiotic by weight; and/or any other appropriate ratio). In some implementations, these ratios may be based on type of individual (e.g., age, gender, etc.), symptoms, underlying cause of symptoms, other associated symptoms, frequency of administration, etc.

In some implementations, a hydrating agent may be utilized with the OAB composition. For example, the OAB composition may be dissolved, mixed, and/or otherwise combined with a hydrating agent. The hydrating agent may include water, juice, milk, alcoholic beverage, sauce, soup, and/or any other appropriate hydrating agent. The hydrating agent selected for use with the OAB composition may be based on the type of OAB composition. For example, a beverage with alcohol that may kill at least a portion of the bacteria in the OAB composition may be used as a hydrating agent for an OAB composition that does not include probiotics (e.g., an OAB composition that includes calcium and/or magnesium) and may not be used as a hydrating agent for an OAB composition that does include probiotics. Water may be used as a hydrating agent for an OAB composition that includes probiotics (e.g., to revive at least a portion of the dried bacteria in the probiotics, if present). In some implementations, fluids present in the mouth of an individual and/or the gastrointestinal tract of an individual may at least partially revive at least a portion of the dried probiotic in the OAB composition.

Applying the hydrating agent may revive organisms in the probiotic, in some implementations. By decreasing the amount of time between hydrating a dry probiotic in the OAB composition and administering the OAB composition, the amount of live probiotic administered may be increased, in some implementations. Thus, in some implementations, the OAB composition may include dried probiotics that are revived (e.g., by a user's application of a hydrating agent) prior to and/or during use.

In some implementations, the OAB composition that includes probiotics may be stored in an at least partially enclosed container. Enclosing the probiotic OAB composition may inhibit sunlight, moisture, and/or air exposure for the probiotic and/or increase the life of the probiotic (e.g., decrease the number of dead probiotic organisms prior to use when compared with unenclosed probiotic). The OAB composition may be enclosed in a removable package. For example, the OAB composition may be disposed in a removable package, such as a jar with a removable lid, individually foil-wrapped capsules and/or individually foil-wrapped tablets.

In some implementations, to increase shelf life and/or to increase the amount of live bacteria that may be administered to an individual, during use, the OAB composition (e.g., the OAB composition that includes probiotics) may be stored in a predetermined temperature range (e.g., in a refrigerator or chiller). In some implementations, the OAB composition with probiotics may be shelf stable at room temperature in predetermined temperatures (e.g., 60-80 degrees Fahrenheit).

In some implementations, the OAB composition (e.g., container including OAB composition) may be maintained at a first temperature range during manufacturing, sale, and/or resale (e.g., refrigerated). In some implementations, the OAB composition may be maintained at a second temperature range by an individual. The second temperature range may be different or the same as the first temperature range. In some implementations, the OAB composition may be refrigerated prior to sale and stored at room temperature by users. Maintaining the OAB composition at the second temperature range may or may not substantially impact the amount of live probiotic administered to an individual.

The OAB composition may be provided with a date of expiration, in some implementations. Use of the OAB composition that includes probiotics after the provided expiration date may cause administration of less than a predetermined minimum amount of probiotic.

The OAB composition is a supplement that is orally administered. The OAB composition may be granular. For example, the OAB composition may be a powder. The OAB composition may resemble salt (e.g., approximately 0.3 microns - approximately 0.5 microns). The OAB composition may be a dissolvable in a liquid (e.g., water, milk, or other beverage). In some implementations, the OAB composition may be applied (e.g., sprinkled, dusted, coated, etc.) to a food.

In some implementations, the OAB composition may be provided to an individual in a fast melting tablet (e.g., produced by crystalline transition, phase transition, sublimation, spray drying, and direct compression). By providing the OAB composition in a fast melting tablet, the tablet may be taken contemporaneously with a meal (e.g., which may be more convenient for individuals, may increase the effectiveness since meals may be less planned, etc.) rather than a large period of time before a meal (e.g. to allow time for a pill to dissolve in the gastrointestinal tract).

In some implementations, the OAB composition may be provided in pre-dosed therapeutically effective amounts. For example, a therapeutically effective amount of the OAB composition may be individually packaged in a container (e.g., foil packet). A therapeutically effective amount of the OAB composition may be provided in a tablet (e.g., pill, fast melt tablet, gel-capsule, etc.).

In some implementations, one or more dosages of the OAB composition may be provided to and/or administered to a user. A user may be administered (e.g., self administered or administered by another individual) one or more dosages of OAB composition in a time frame (e.g., definite or indefinite time period, such as daily, weekly, for 1 week, and/or for 1 year). In some implementations, a user may be administered more than one dosage at a given time (e.g., 2 pills with a meal, approximately 2 dosages to approximately 4 dosages per day). In some implementations, an individual may be administered the OAB composition at least twice a day. For example, an individual may be administered the OAB composition four times a day. An individual's dosage may be based on underlying user conditions (e.g., diseases, disorders, weight, diet, etc.).

A dosage of the OAB composition may include approximately 100 mg to approximately 2500 mg of calcium. For example, a dosage of the OAB composition may be approximately 500 mg to approximately 1200 mg of calcium. A dosage of the OAB composition may include approximately 50 mg to approximately 1500 mg of magnesium. In some implementations, the amount of magnesium in a dosage of the OAB composition may be selected to produce a laxative effect in individuals and may be approximately 800 mg to approximately 1200 mg of magnesium. In some implementations, a dosage of the OAB composition may include approximately 1000 mg to approximately 1200 mg calcium and approximately 600 to approximately 800 mg of magnesium.

In some implementations, a dosage of the OAB composition may include approximately 800 mg to approximately 1000 mg of Calcium and approximately 200 to approximately 300 mg of Magnesium. For example, a dosage of the OAB may include approximately 800-900 mg of Calcium and approximately 200-300 mg of Magnesium. The OAB may include probiotics, such as *Oxalobacter formigenes.* The OAB may include approximately 100 mg to approximately 300 mg of probiotic and/or any other appropriate amount.

In some implementations, the OAB composition may be provided in a container and a user may measure a therapeutically effective amount from the container. For example, the OAB composition may be provided in a jar and an individual may use a measuring device (e.g., measuring spoon, scoop, cup, scale, or other appropriate measuring device) to measure for administration a therapeutically effective amount. In some implementations, the OAB composition may be provided in a shaker (with the ability to dose quantities and/or without the ability to does quantities) to allow a user to shake the OAB composition on food and/or into a hydrating agent.

In some implementations, the OAB composition may be orally administered concurrently with the consumption of food (e.g., meat, vegetables, beverages, and/or combinations thereof). For example, the OAB composition may be administered (e.g., consumed) prior to eating (e.g., 1 hour prior to eating, 15 minutes prior to eating, and/or other appropriate times). The OAB composition may be administered during consumption of foods. For example, the OAB composition may be sprinkled onto food, cooked into foods (e.g., to allow concurrent consumption), drunk during a meal, etc. In some implementations, the OAB composition may be administered immediately after consuming food (e.g., within 10 minutes of a meal, etc.). In some implementations, the OAB composition may be administered during a time period that allows the OAB composition to interact with the food prior to all of the oxalate in the food being absorbed by the body of the individual.

In some implementations, consumption of the OAB composition during meals that are not high in oxalate may not substantially negatively impact an individual's health.

In some implementations, an individual may have a condition in which a reduction of oxalate absorption from consumed foods may provide improved health. For example, an individual may have fibromyalgia. A reduction in the amount of oxalate absorbed from consumed foods may reduce pain occurrences and/or reduce the severity of pain occurrences. Thus, the OAB composition may be administered to the individual in a therapeutically effective amount. The administration of the OAB composition may reduce the amount of oxalate absorbed from consumed foods (e.g., by degrading oxalate, binding with oxalate, and/or interfering with oxalate absorption in the body). The OAB composition may be administered concurrently with the consumption of food (e.g., meals, snacks, beverages, etc.).

In some implementations, an individual may have a diet rich in oxalate. For example, foods such as greens (e.g., spinach and beet greens), rhubarb, beets, nuts, chocolate, tea, coffee, soybeans, and strawberries may have high levels of oxalate. A diet rich in these high oxalate foods may increase levels of oxalate absorption in the individual. Increasing the amount of oxalate absorbed by an individual's body may cause and/or increase the risk for negative health effects. The OAB composition may be administered in a therapeutically effective amount to reduce the amount of oxalate absorbed by the body (e.g., when compared to the amount of oxalate absorbed by a body without the administration of the OAB composition). Thus, the OAB composition may reduce and/or inhibit the negative health effects associated with high oxalate absorption in the body.

In some implementations, an individual may have kidney stones and/or have a predisposition to kidney stones. Following a low oxalate diet may be difficult and/or cause nutritional deficiencies since many oxalate rich foods may also be healthful. Thus, the OAB composition may be administered to reduce the oxalate absorption from consumed foods. The OAB composition may be administered to an individual in a therapeutically effective amount. By administering the OAB composition in the individual, the amount of oxalate absorption from consumed foods may be reduced.

In some implementations, antibiotics may be administered to an individual. Antibiotics may affect the flora of the gastrointestinal tract. An individual may select an OAB composition that includes probiotics to promote a healthy flora in the gastrointestinal tract of the individual. Administration of the OAB composition may reduce the amount of oxalate absorbed from consumed foods and/or increase the healthy flora in the individual's gastrointestinal tract or portions thereof.

In some implementations, an individual may have pain due to oxalate absorption (e.g., fibromyalgia, vulvodynia, arthritis, calcification of spine and/or other regions of the body, such as the shoulder joint). The OAB composition may be administered in a therapeutically effective amount to the individual. The therapeutically effective amount of the OAB composition may be an amount of the OAB composition that when administered reduces, stops, and/or inhibits pain. For example, pain in an individual in treatment may be monitored using a standardized pain levels, such as a comparative pain scale.

In some implementations, individuals may have health diseases and/or disorders in which a restrictive diet, such as a low oxalate diet, high fiber, and/or low fat diet has been prescribed. When the restrictive diet is based on decreasing levels of oxalate absorbed by the body, the OAB composition may be administered instead of and/or in conjunction with the restrictive diet. An individual may obtain results equal to and/or better than restrictive diet alone with administration of the OAB composition. The effect of the OAB may be measured using urine and/or blood tests to measure oxalate levels in the body. In some implementations, the effects of the administration of the OAB composition may be quicker than obtaining results of the restrictive diet alone.

In some implementations, the OAB composition may be administered to an individual to improve the health of the individual. For example, the individual may have a predisposition to hyper-absorption of oxalate, hyperaoxaluria, and/or may have pain (e.g., due to fibromyalgia and/or rheumatoid arthritis). The OAB composition may administered at least two times a day. In some implementations, the OAB composition may be administered proximate (e.g., within approximately 15 minutes of meals) consumption of meals (e.g., food and/or drinks). The effectiveness of the OAB composition may be evaluated by analysis of symptoms (e.g., pain) associated with an individuals disease and/or levels of oxlate in the individual (e.g., via blood and/or urine tests).

Figure 1 illustrates an implementation of a process 100 for administering the OAB composition. A therapeutically effective amount of the OAB composition may be administered to an individual (operation 110). For example, the OAB composition may be orally administered to the individual. The OAB composition may include dolomite. An amount of oxalate absorbed from food consumed by the individual may be reduced (operation 120). For example, if food that includes oxalate, such as chips and spinach dip and/or a strawberry smoothie, is consumed by the individual, the amount of oxalate absorbed from the food may be decreased by the administered OAB composition. For example, the calcium and/or magnesium in the OAB composition may decrease the amount of oxalate absorbed by the body (e.g., by coupling with the oxalate in the food and/or inhibiting absorption of the oxalate from the food). In some implementations, when the OAB composition includes probiotics, the probiotics in the OAB composition may decrease the amount of oxalate absorbed by the body (e.g., by degrading the oxalate and/or inhibiting absorption of the oxalate from the food).

Process 100 may be implemented with various OAB compositions as described herein. In addition, various operations may be added, deleted, and/or modified. In some implementations, process 100 may be performed in combination with other processes and/or operations. For example, the type of OAB composition (e.g., an OAB composition including dolomite, ratio of calcium to magnesium, inclusion of probiotics, etc.) may be selected. In some implementations, the type of OAB composition selected may be based on the severity of an individual's symptoms, the type of symptoms in an individual, cost, speed of recovery, etc. In some implementations, the OAB composition may be administered orally. The OAB composition may be administered concurrently with the consumption of food, in some implementations. For example, the OAB composition may be administered 15 minutes or less before consumption of food.

In some implementations, the OAB composition may be applied to (e.g., sprinkled, spread, dusted, mixed, dissolved in, cooked with, etc.) the food to be consumed. The OAB composition may thus be administered with the food to be consumed.

In some implementations, a level of oxalate in the individual's body (e.g., in the urine and/or blood) and/or an individual's symptoms may be monitored and a determination may be made whether to alter the predetermined therapeutically effective amount of OAB composition to be administered to the individual. For example, the next dosage of the OAB composition to be administered to the individual as a therapeutically effective amount may be altered if after consumption of an initial series of the OAB composition (e.g., one or more administrations of the OAB composition) the measured (e.g., from the monitoring) level of oxalate in the individual's body is greater than a predetermined healthy level of oxalate. In some implementations, the amount of the therapeutically effective dosage to be administered in the next dosage may not be altered based on the measurement of the oxalate levels (e.g., from monitoring the oxalate level in the body). For example, if the level of oxalate in the body is reduced, then the quantity of the therapeutically effective amount may not be altered. In some implementations, if the levels of oxalate in the body are reduced, the quantity of the therapeutically effective amount may be reduced and/or administration of the OAB composition may be less frequent and/or stopped.

In some implementations, an individual may take a first therapeutically effective amount of the OAB composition on a first schedule (e.g., daily, with every meal, etc.). When an individual exhibits symptoms associated with excess oxalate absorption (e.g., pain, kidney stones, gout, etc.), the individual may take a second therapeutically effective amount of the OAB composition on a second schedule. The second therapeutically effective amount of the OAB composition may be greater than or equal to the first therapeutically effective amount of the OAB composition . In some implementations, the second schedule may be the same as the first schedule and/or different (e.g., more frequent). In some implementations, when the symptoms associated with the excess oxalate absorption are reduced and/or inhibited (e.g., by the OAB composition), the administration of the OAB composition may be modified to the first therapeutically effective amount on the first schedule.

Individuals have been described. In some implementations, an individual may include a human or other mammal. Although administration has been described as to individuals, a plurality of individuals may be administered the OAB composition via direct application to consumables (e.g., foods and/or beverages).

Administration of the OAB composition proximate meals has been described in some implementations. A meal may include a food and/or beverage. The quantity of food and/or beverage consumed in the meal may be large (e.g., a heavy meal) or small (e.g., a snack such as a few blueberries or small quantity of coffee). The administration of the OAB composition proximate a meal may include administration before, during, and/or after a meal. For example, the OAB composition may be administered 1 hour before a meal, at least 5 minutes prior to a meal, and/or immediately prior to a meal. In some implementations, the OAB composition may be administered within 15 minutes and/or within 30 minutes of consuming a meal.

It is to be understood the implementations are not limited to particular systems or processes described which may, of course, vary. It is also to be understood that the terminology used herein is for the purpose of describing particular implementations only, and is not intended to be limiting. As used in this specification, the singular forms "a", "an" and "the" include plural referents unless the content clearly indicates otherwise. Thus, for example, reference to "a carbonate" includes a combination of two or more carbonate OAB compositions and reference to "a probiotic" includes different types and/or combinations of probiotics. In addition, reference to "calcium" includes different types, forms, and/or combinations of OAB compositions including the element calcium.

## Claims

1. An oxalate absorption blocking composition comprising 100 mg to 2500 mg calcium and 50 mg to 1500 mg magnesium for use in reducing fibromyalgia pain in a mammal and/or reducing pain due to calcification of the spine and/or a shoulder of a mammal, wherein a therapeutically effective amount of the oxalate absorption blocking composition is for oral administration.

2. The oxalate absorption blocking composition for use according to claim 1, wherein the oxalate absorption blocking composition comprises one or more probiotics.

3. The oxalate absorption blocking composition for use according to claim 1 or claim 2, wherein the oxalate absorption blocking composition comprises *Oxalobacter formigenes.*

4. The oxalate absorption blocking composition for use according to any preceding claim, wherein an atomic ratio of calcium to magnesium in the oxalate absorption blocking composition comprises 1 to 2 Calcium: to 1 Magnesium.

5. The oxalate absorption blocking composition for use according to any preceding claim, wherein the oxalate absorption blocking composition is for administration at least twice a day.

6. The oxalate absorption blocking composition for use according to any preceding claim, wherein the oxalate absorption blocking composition is for administration proximate consumption of a meal.

7. The oxalate absorption blocking composition for use according to any preceding claim, wherein the oxalate absorption blocking composition comprises 800 mg of Calcium to 1000 mg of Calcium, and wherein the oxalate absorption blocking composition comprises 200 mg of Magnesium to 300 mg of Magnesium.

8. The oxalate absorption blocking composition for use according to any preceding claim, wherein the oxalate absorption blocking composition further comprises an analgesic.

9. The oxalate absorption blocking composition for use according to any preceding claim, wherein the oxalate absorption blocking composition comprises 800 mg of Calcium to 1000 mg of Calcium.

10. The oxalate absorption blocking composition for use according to any preceding claim, wherein the oxalate absorption blocking composition comprises 200 mg of Magnesium to 300 mg of Magnesium.

## Patentansprüche

1. Oxalat-Absorptionsblockierungs-Zusammensetzung, umfassend 100 mg bis 2500 mg Calcium und 50 mg bis 1500 mg Magnesium, zur Verwendung bei der Verringerung von Fibromyalgie-Schmerzen bei einem Säugetier und/oder der Verringerung von Schmerzen aufgrund von Verkalkung der Wirbelsäule und/oder einer Schulter eines Säugetiers, wobei eine therapeutisch wirksame Menge der Oxalat-Absorptionsblockierungs-Zusammensetzung zur oralen Verabreichung bestimmt ist.

2. Oxalat-Absorptionsblockierungs-Zusammensetzung zur Verwendung nach Anspruch 1, wobei die Oxalat-Absorptionsblockierungs-Zusammensetzung ein oder mehrere Probiotika umfasst.

3. Oxalat-Absorptionsblockierungs-Zusammensetzung zur Verwendung nach Anspruch 1 oder Anspruch 2, wobei die Oxalat-Absorptionsblockierungs-Zusammensetzung *Oxalobacter formigenes* umfasst.

4. Oxalat-Absorptionsblockierungs-Zusammensetzung zur Verwendung nach einem vorhergehenden Anspruch, wobei ein Atomverhältnis von Calcium zu Magnesium in der Oxalat-Absorptionsblockierungs-Zusammensetzung 1 bis 2 Calcium: zu 1 Magnesium umfasst.

5. Oxalat-Absorptionsblockierungs-Zusammensetzung zur Verwendung nach einem vorhergehenden Anspruch, wobei die Oxalat-Absorptionsblockierungs-Zusammensetzung mindestens zweimal täglich zu verabreichen ist.

6. Oxalat-Absorptionsblockierungs-Zusammensetzung zur Verwendung nach einem vorhergehenden Anspruch, wobei die Oxalat-Absorptionsblockierungs-Zusammensetzung unmittelbar vor oder nach Verzehr einer Mahlzeit zu verabreichen ist.

7. Oxalat-Absorptionsblockierungs-Zusammensetzung zur Verwendung nach einem vorhergehenden Anspruch, wobei die Oxalat-Absorptionsblockierungs-Zusammensetzung 800 mg Calcium bis 1000 mg Calcium umfasst, und wobei die Oxalat-Absorptionsblockierungs-Zusammensetzung 200 mg Magnesium bis 300 mg Magnesium umfasst.

8. Oxalat-Absorptionsblockierungs-Zusammensetzung zur Verwendung nach einem vorhergehenden Anspruch, wobei die Oxalat-Absorptionsblockierungs-Zusammensetzung ferner ein Analgetikum umfasst.

9. Oxalat-Absorptionsblockierungs-Zusammensetzung zur Verwendung nach einem vorhergehenden Anspruch, wobei die Oxalat-Absorptionsblockierungs-Zusammensetzung 800 mg Calcium bis 1000 mg Calcium umfasst.

10. Oxalat-Absorptionsblockierungs-Zusammensetzung zur Verwendung nach einem vorhergehenden Anspruch, wobei die Oxalat-Absorptionsblockierungs-Zusammensetzung 200 mg Magnesium bis 300 mg Magnesium umfasst.

## Revendications

1. Composition de blocage d'absorption d'oxalate comprenant 100 mg à 2 500 mg de calcium et 50 mg à 1 500 mg de magnésium à utiliser pour la réduction de la douleur due à une fibromyalgie chez un mammifère et/ou la réduction de la douleur due à une calcification de la colonne vertébrale et/ou de l'épaule d'un mammifère, une quantité thérapeutiquement efficace de la composition de blocage d'absorption d'oxalate étant destinée à l'administration orale.

2. Composition de blocage d'absorption d'oxalate à utiliser selon la revendication 1, dans laquelle la composition de blocage d'absorption d'oxalate comprend un ou plusieurs probiotiques.

3. Composition de blocage d'absorption d'oxalate à utiliser selon la revendication 1 ou la revendication 2, dans laquelle la composition de blocage d'absorption d'oxalate comprend de l'*Oxalobacter formigenes.*

4. Composition de blocage d'absorption d'oxalate à utiliser selon l'une quelconque des revendications précédentes, dans laquelle un rapport atomique de calcium au magnésium dans la composition de blocage d'absorption d'oxalate comprend 1 à 2 calcium : pour 1 magnésium.

5. Composition de blocage d'absorption d'oxalate à utiliser selon l'une quelconque des revendications précédentes, dans laquelle la composition de blocage d'absorption d'oxalate est destinée à être administrée au moins deux fois par jour.

6. Composition de blocage d'absorption d'oxalate à utiliser selon l'une quelconque des revendications précédentes, dans laquelle la composition de blocage d'absorption d'oxalate est destinée à être administrée au moment de la consommation d'un repas.

7. Composition de blocage d'absorption d'oxalate à utiliser selon l'une quelconque des revendications précédentes, dans laquelle la composition de blocage d'absorption d'oxalate comprend 800 mg de calcium à 1 000 mg de calcium, et dans laquelle la composition de blocage d'absorption d'oxalate comprend 200 mg de magnésium à 300 mg de magnésium.

8. Composition de blocage d'absorption d'oxalate à utiliser selon l'une quelconque des revendications précédentes, dans laquelle la composition de blocage d'absorption d'oxalate comprend en outre un analgésique.

9. Composition de blocage d'absorption d'oxalate à utiliser selon l'une quelconque des revendications précédentes, dans laquelle la composition de blocage d'absorption d'oxalate comprend 800 mg de calcium à 1 000 mg de calcium.

10. Composition de blocage d'absorption d'oxalate à utiliser selon l'une quelconque des revendications précédentes, dans laquelle la composition de blocage d'absorption d'oxalate comprend 200 mg de magnésium à 300 mg de magnésium.
